# EUROPÄISCHE PATENTSCHRIFT

(11) **EP 2 192 102 B1**
(45) Veröffentlichungstag und Bekanntmachung des Hinweises auf die Patenterteilung: **25.12.2013**
(21) Anmeldenummer: 09014387.6
(22) Anmeldetag: 18.11.2009
(51) Int. Cl.: C07C 45/29

(54) **Verfahren zur Herstellung von wässrigen Formaldehyd-Lösungen**
Method for producing aqueous formaldehyde solutions
Procédé de fabrication de solutions d'acide de polyacrylique aqueux

(30) Priorität: 29.11.2008 DE 102008059701
(43) Veröffentlichungstag der Anmeldung: 02.06.2010
(73) Patentinhaber: Bayer Intellectual Property GmbH, 40789 Monheim (DE)
(72) Erfinder: Müller, Heinz-Herbert, Dr., 47809 Krefeld (DE); Ehlers, Stephan, Dr., 40477 Düsseldorf (DE); Schlemenat, Andreas, Dr., 50937 Köln (DE); Werner, Arnulf, Dr., 41540 Dormagen (DE); Schiffhauer, Martin, 40699 Erkrath (DE)
(74) Vertreter: BIP Patents

(56) Entgegenhaltungen:
- MAIWALD M ET AL: "Quantitative high-resolution on-line NMR spectroscopy in reaction and process monitoring" JOURNAL OF MAGNETIC RESONANCE, ACADEMIC PRESS, ORLANDO, FL, US, Bd. 166, Nr. 2, 1. Februar 2004 (2004-02-01), Seiten 135-146, XP004483621 ISSN: 1090-7807
- BURCHAM L J ET AL: "The Origin of the Ligand Effect in Metal Oxide Catalysts: Novel Fixed-Bed in Situ Infrared and Kinetic Studies during Methanol Oxidation" JOURNAL OF CATALYSIS, ACADEMIC PRESS, DULUTH, MN, US, Bd. 203, Nr. 1, 1. Oktober 2001 (2001-10-01), Seiten 104-121, XP004432431 ISSN: 0021-9517
- J. WORKMANN, JR: J. NEAR INFRARED SPECTROSC., Bd. 1, 1993, Seiten 221-245, XP002574582
- OMAR YÉPEZ ET AL: 'Electrochemical Oxidation of Methanol at Hydrogen-Loaded PdPtRu-Coated Pd Electrodes' ELECTROCHEMICAL AND SOLID-STATE LETTERS Bd. 8, Nr. 3, 01 Januar 2005, Seiten E35 - E38, XP055005393 DOI: 10.1149/1.1857112 ISSN: 1099-0062
- J. Böcker: "Spektroskopie", 1997, Vogel Buchverlag
- G. Reuss, W. Disteldorf, O. Grundler, A. Hilt: "Ullmann's Encyclopedia of Industrial Chemistry 5th Edition Vol. A 11", 1988, VCH Verlagsgesellschaft
- H. R. Gerberich, G. C. Seaman: "Kirk-Othmer Encyclopedia of Chemical Technology 4th Edition Vol. 11", 1994, Wiley Interscience

## Beschreibung

Die Erfindung betrifft ein Verfahren zur Herstellung von wässrigen Formaldehyd-Lösungen, bei dem der Formaldehyd-Gehalt der bei der Formaldehyd-Synthese anfallenden wässrigen Formaldehyd-Lösungen mit einem Online-Analysenverfahren ausgewählt aus Nah-Infrarot-, Mittel-Infrarot- und Raman-Spektroskopie bestimmt wird, wobei das Spektrum in ein chemometrisches Kalibrationsmodell eingegeben wird, und der gewünschte Formaldehyd-Gehalt anschließend durch Wasserzugabe eingestellt werden kann, so dass eine wässrige Formaldehyd-Lösung mit definiertem Formaldehyd-Gehalt (Formalin) erhalten wird.

Formaldehyd gehört zu den wichtigsten organischen Grundstoffen in der chemischen Industrie und dient als Ausgangsstoff für polymere Harze (Harnstoff-Formaldehyd-Harze, Melamin-Formaldehyd-Harze, Phenol-Harze), für Polyacetale und für diverse organisch-chemische Produkte (z. B. Pentaerythrit, Trimethylolpropan, Neopentylglykol, Methylendianilin, Hexamethylentetramin, Ethylendiamintetraessigsäure, Nitrilotriessigsäure). Neben der Verwendung als Ausgangsstoff für chemische Produkte wird Formaldehyd in Form von wässrigen Lösungen als Desinfektions - und Konservierungsmittel eingesetzt, z. B. in der Kosmetikindustrie.

Großtechnisch wird Formaldehyd durch partielle Oxidation von Methanol hergestellt:

CH₃OH + 0,5 O₂ → CH₂O + H₂O

Die wichtigsten Verfahren sind:
(1) Partielle Oxidation und Dehydrierung in Gegenwart von kristallinem Silber mit einem Unterschuss an Luft bei 680 - 720°C bei einem Methanol-Umsatz von 97 - 98%
(2) Partielle Oxidation und Dehydrierung in Gegenwart von kristallinem Silber oder Silbernetzen mit einem Unterschuss an Luft bei 600 - 650°C bei einem Methanol-Umsatz von 77 - 87 % und anschließender destillativer Rückgewinnung des Methanols und Rückführung in den Prozess
(3) Oxidation mit Eisenoxid / Molybdänoxid-Katalysatoren mit einem Überschuss an Luft bei einem Methanol-Umsatz von 98 - 99 % (sog. Formox-Verfahren)

Die Verfahren sind beschrieben in G. Reuss, W. Disteldorf, O. Grundler, A. Hilt "Formaldehyde" in "Ullmann's Encyclopedia of Industrial Chemistry" 5 th Edition, Vol. A 11, Seiten 624 - 633 ; VCH Verlagsgesellschaft Weinheim 1988 und in H. R. Gerberich, G. C. Seaman "Formaldehyde" in "Kirk-Othmer Encyclopedia of Chemical Technology", 4 th Edition, Vol. 11, Seiten 935 - 939, Wiley Interscience, New York 1994.

JOURNAL OF MAGNETIC RESONANCE, Bd. 166, Nr. 2, 1. Februar 2004 (2004-02-01), Seiten 135-146 offenbart ein Verfahren, in dem durch online NMR Spektroskopie die Konzentration einer Formaldehyd-Lösung bestimmt werden kann.

Electrochemical and Solid-State Letters, Bd. 8, Nr. 3, 2005, Seiten E35-E38 offenbart die diskontinuierliche Herstellung von Formaldehyd durch elektrochemische Oxidation von Methanol und Bestimmung des produzierten Formaldehyds nach jedem Experiment durch UV-VIS-NIR-Spektroskopie.

Bei den unterschiedlichen Herstellverfahren fällt der Formaldehyd im Prozess jeweils als wässrige Lösung mit einer Konzentration von üblicherweise 25 - 56 Gew.-%, bezogen auf das Gewicht der Lösung, an.

Sowohl bei den Silberkontakt-Verfahren wie auch bei den Metalloxid-Kontakt-Verfahren werden die heißen Reaktionsgase, die im Wesentlichen aus dem gebildeten Formaldehyd, nicht umgesetztem Methanol, Wasser, CO₂, CO, CH₄, Sauerstoff, Wasserstoff und Stickstoff bestehen, nach der Reaktion abgekühlt. Anschließend wird der entstandene Formaldehyd mit Wasser oder verdünnter Formaldehyd-Lösung ausgewaschen. Die Wäsche erfolgt üblicherweise mehrstufig in einer Absorptionskolonne im Gegenstrom. Die freiwerdende Absorptionswärme wird über einen Produktkreislauf in Wärmetauschern abgeführt und kann an anderer Stelle zur Beheizung eingesetzt werden.

Die Waschflüssigkeit wird im Allgemeinen auf dem Kopf der letzten Absorptionsstufe aufgegeben. Über die Menge an Waschflüssigkeit kann die Formaldehyd-Konzentration grob eingestellt werden.

Beim Verfahren mit vollständigem Methanol-Umsatz erhält man in der ersten Absorptionsstufe üblicherweise eine Formaldehyd-Lösung mit ca. 40 - 42 Gew.-%, bezogen auf das Gewicht der Lösung. In den weiteren Absorptionsstufen ist die Formaldehyd-Konzentration geringer.

Die wässrigen Formaldehyd-Lösungen erhalten noch Restmengen an Methanol (ca. 1 Gew.% beim Verfahren mit vollständigem Methanol-Umsatz). Beim Verfahren mit unvollständigem Methanol-Umsatz beträgt der Methanol-Gehalt der Absorptionslösung üblicherweise 5 - 15 Gew.%. Zur Abtrennung des Methanols wird die Methanol-haltige Formaldehyd-Lösung abschließend destilliert. Man erhält dann üblicherweise Formaldehyd-Lösungen mit einer Konzentration von ca. 50 Gew.% (45 - 55 Gew.-%) und 0,5 - 1 Gew.% Methanol.

Beim Formox-Verfahren fallen in Abhängigkeit von der Menge an zugegebenem Waschwasser Formalin-Lösungen mit einer Konzentration von 37 - 55 Gew.-% Formaldehyd und 0,5 - 1,5 Gew.-% Methanol an. Dabei sind die Konzentrationsangaben in Gew.-% jeweils bezogen auf das Gewicht der wässrigen Formaldehyd-Lösung.

Formaldehyd kommt als wässrige Lösung (sog. "Formalin") in den Handel, wobei Formalin mit unterschiedlichen Konzentrationen im Bereich von 30 - 56 Gew.-% angeboten wird. Der am weitesten verbreitete Typ ist Formalin mit einer Formaldehyd-Konzentration von 37 Gew.-%. Größere Bedeutung haben auch Formalin-Lösungen mit 32 Gew.-% Formaldehyd, die eine bessere Lagerstabilität aufweisen.

Wie bei Handelsprodukten üblich, wird die Qualität der Formalin-Typen vom Hersteller über eine Spezifikation beschrieben und dem Kunden gegenüber garantiert. Als Spezifikationsmerkmale werden für Formalin üblicherweise der Gehalt an Formaldehyd, der Gehalt an Methanol und die Farbe, z. B. als Hazen-Farbzahl, teilweise auch der Gehalt an Acidität bzw. Ameisensäure angegeben. Der in der Spezifikation ausgewiesene Gehalt an Formaldehyd, der üblicherweise in einem Bereich von + / - 0,25 Gew.-% angegeben wird, ist für den Kunden naturgemäß eine sehr wichtige Größe. Der Kunde prüft bei seiner Wareneingangskontrolle, dass der Formaldehyd-Gehalt der Formalin-Lieferung im Spezifikationsbereich liegt, insbesondere, dass die Untergrenze des Formaldehyd-Gehaltes nicht unterschritten wird, weil er sonst Wasser statt Wertstoff bezahlen würde.

Für den Formalin-Hersteller bedeutet das, dass streng darauf zu achten ist, bei der Formalin-Auslieferung den in der Spezifikation angegebenen Formaldehyd-Gehalt genau einzuhalten.

Bei der Herstellung der wässrigen Formaldehyd-Lösungen kann die Formaldehyd-Konzentration aber im Allgemeinen nur relativ grob eingestellt und eingehalten werden.

Stand der Technik ist es daher, dass die Formalin-Produktion letztlich praktisch batchweise erfolgt, indem eine Menge der wässrigen Formaldehyd-Lösung mit höherer Konzentration produziert und in einen Behälter gegeben wird. Dann wird der Formaldehyd-Gehalt der im Behälter befindlichen Formaldehyd-Lösung analytisch bestimmt. Üblicherweise wird hierzu eine repräsentative Probe entnommen und im Labor der Formaldehyd-Gehalt nach Standard-Verfahren analysiert. Üblicherweise erfolgt die Analyse des Formaldehyd-Gehaltes durch Titration nach der sog. "Natriumsulfit-Methode" (J. F. Walker, Formaldehyde, Reinhold Publishing Corp., New York 1964, S. 486).

Nach der Ermittlung des Formaldehyd-Gehaltes wird die Menge an Wasser in den Behälter gegeben, die notwendig ist, um die im Behälter vorhandene Menge an Formaldehyd-Lösung auf die vorgegebene Konzentration (Sollkonzentration) an Formaldehyd zu bringen.

Dieses praktizierte Verfahren ist umständlich und in Hinblick auf Probenahme und Laboranalysen aufwendig und arbeitsintensiv. Nachteilig ist, dass das Verfahren zeitraubend ist, weil es aufgrund der erforderlichen Laboranalysen relativ lange dauert, bis die Einstellung der Konzentration abgeschlossen ist. Nachteilig ist, dass geringere Schwankungen im Produktionsprozess nicht immer erfasst werden. Da die Konzentrationseinstellung batchweise erfolgt, sind letztlich mehrere Behälter notwendig, die mit Investitions - und Betriebskosten verbunden sind. Das praktizierte Verfahren weist also erhebliche Nachteile auf.

Für die Herstellung von wässrigen Formaldehyd-Lösungen mit genau eingestellter Konzentration wäre es wünschenswert, den Formaldehyd-Gehalt direkt durch Online-Analyse in der Absorberlösung, d.h. in Wasser bzw. in den wässrigen Formaldehyd-Lösungen, zu messen. Es hat nicht an Versuchen gefehlt, entsprechende Messverfahren zu entwickeln, z. B. durch Messung der Dichte oder des Brechungsindex. Eine wichtige Voraussetzung für die Online-Analytik ist, dass das Online-Messverfahren für die Bestimmung des Formaldehyd-Gehaltes eine Genauigkeit erreicht, die besser als die Abweichung vom Formaldehyd-Mittelwert ist, der in der Spezifikation angegeben ist. Insbesondere muss das Online-Messverfahren in der Lage sein, den Formaldehyd-Gehalt mit hoher Richtigkeit, d. h. ohne merklichen systematischen Fehler, in Gegenwart von Methanol auch bei sich ändernden Methanol-Konzentrationen zu bestimmen. Messungen des Brechungsindex und der Dichte scheiden als Online-Verfahren aus, weil die Messungen letztlich nicht selektiv für den Formaldhyd - und den Wasser-Gehalt sind, sondern die gemessene Größe auch vom Methanol-Gehalt beeinflusst wird. Schwankungen im Methanol-Gehalt, die im Herstellprozess auftreten können, würden dementsprechend ggf. zu Fehlern beim gemessenen Formaldehyd-Gehalt führen, die nicht toleriert werden können.

Zur quantitativen Analyse der Zusammensetzung von Stoffgemischen sind grundsätzlich spektroskopische Verfahren bekannt, beispielsweise Nahinfrarot-(N)R)-Spektroskopie, Mittelinfrarot-Spektroskopie und Raman-Spektroskopie. Das analytische Verfahren Nahinfrarot-(NIR)-Spektroskopie ist eine weit verbreitete Technik, die sowohl im Laborbereich als auch im Online-Betrieb eingesetzt wird [J. Workman; "A review of process near infrared spectroscopy" ; J. Near Infrared Spectroscopy 1,221 -245 (1993)].

Die Vorteile der Kombination von NIR-Spektroskopie mit Lichtwellenleitern gegenüber dem Einsatz der Mittel-Infrarot-Spektroskopie sind bekannt aus Khettry "Inline Monitoring of Polymeric Processes ", Antec 92, 2674 - 2676.

Für quantitative Analysen wird die NIR-Spektroskopie häufig in Kombination mit chemometrischen Auswertemethoden verwendet. Gebräuchlich ist dabei z. B. das Least-Square-Verfahren, wie z. B. beschrieben in C. Miller "Chemometrics for online spectroscopy aplications - theory and practice", J. Chemometrics 14, 513 - 528 (2000) oder "Multivariate Analysis of Near Infrared Spectra Using G-Programming Language", J. Chem. Inf. Comput. Sci. 40, 1093 - 1100 (2000). Einen Überblick über die Verwendung von multivarianten chemometrischen Kalibrationsmodellen in der analytischen Chemie gibt ferner "Multivariate Kalibration", J.-P. Conzen; 2001, ISBN 3-929431-13-0.

Die Verwendung von NIR-Techniken für spezielle Messaufgaben ist ferner bekannt aus:

WO-A- 2002051898 (Produktionskontrolle bei der Herstellung von wässrigen Formaldehyd-Harzen), BR 200302120 (NIR-Spektroskopie bei der Herstellung von Verbundstoff-Paneelen aus Formaldehyd-Harzen), E: Dessipiri; Europ. Polym. J. 39, 1533 - 1540 (2003) (Online-NIR-Spektroskopie bei der Herstellung von Formaldehyd-Harzen).

Im Stand der Technik ist die Möglichkeit des Einsatzes der genannten spektroskopischen Verfahren zur online-Konzentrationsbestimmung von Formaldehyd in wässrigen Lösungen enthaltend Formaldehyd und Methanol bei schwankenden Methanol-Gehalten jedoch nicht bekannt. Nach dem Stand der Technik werden solche spektroskopischen Verfahren zur quantitativen Bestimmung des Formaldehyd-Gehaltes bei der Herstellung von wässrigen Formaldehyd-Lösungen entsprechend ebenfalls nicht eingesetzt.

Die Aufgabe der vorliegenden Erfindung ist die Bereitstellung eines einfachen und wirtschaftlichen kontinuierlichen Verfahrens zur Herstellung von wässrigen Formaldehyd-Lösungen definierter Formaldehyd-Konzentration, bei dem die aufwendige Probennahme und Labor-Analytik entfallen kann.

Die Erfindung betrifft ein Verfahren zur kontinuierlichen Herstellung einer wässrigen Formaldehyd-Lösung mit einem Formaldehyd-Gehalt von 25 - 56 Gew.-%, bezogen auf das Gewicht der wässrigen Formaldehyd-Lösung, bei dem
a) Formaldehyd durch Oxidation von Methanol hergestellt wird und als wässrige Formaldehyd-Lösung erhalten wird, und
b) der Formaldehyd-Gehalt der wässrigen Formaldehyd-Lösung durch Messung eines Nah-Infrarot-, Mittel-Infrarot oder Raman-Spektrums wenigstens 1mal pro Minute (Online-Analyse) ermittelt wird, wobei das Spektrum in ein chemometrisches Kalibrationsmodell eingeben wird, und
c) der ermittelte Formaldehyd-Gehalt mit einem vorgegebenen Sollwert verglichen wird, und, wenn der ermittelte Formaldehyd-Gehalt größer ist als der vorgegebene Sollwert für den Formaldehydgehalt,
d) der gewünschte Formaldehyd-Gehalt der wässrigen Formaldehyd-Lösung durch Zugabe von Wasser eingestellt wird.

Die Herstellung der wässrigen Formaldehyd-Lösung in Schritt a) kann nach einem der aus dem Stand der Technik bekannten Verfahren erfolgen. Beispielhaft sei auf die wichtigsten Verfahren nochmals hingewiesen. Dies sind:
(1) Partielle Oxidation und Dehydrierung in Gegenwart von kristallinem Silber mit einem Unterschuss an Luft bei 680 - 720°C bei einem Methanol-Umsatz von 97 - 98%
(2) Partielle Oxidation und Dehydrierung in Gegenwart von kristallinem Silber oder Silbernetzen mit einem Unterschuss an Luft bei 600 - 650°C bei einem Methanol-Umsatz von 77 - 87 % und anschließender destillativer Rückgewinnung des Methanols und Rückführung in den Prozess
(3) Oxidation mit Eisenoxid / Molybdänoxid-Katalysatoren mit einem Überschuss an Luft bei einem Methanol-Umsatz von 98 - 99 % (sog. Formox-Verfahren)

Die Verfahren sind beschrieben in G. Reuss, W. Disteldorf, O. Grundler, A. Hilt "Formaldehyde" in "Ullmann's Encyclopedia of Industrial Chemistry" 5 th Edition, Vol. A 11, 624 - 633 ; VCH Verlagsgesellschaft Weinheim 1988 und in H. R. Gerberich, G. C. Seaman "Formaldehyde" in "Kirk-Othmer Encyclopedia of Chemical Technology", 4 th Edition, Vol. 11, 935 - 939, Wiley Interscience, New York 1994.

Bei den unterschiedlichen Herstellverfahren fällt der Formaldehyd im Prozess jeweils als wässrige Lösung mit einer Konzentration von üblicherweise 25 - 56 Gew.-%, bevorzugt 30 - 50 Gew.-%, bezogen auf das Gewicht der Lösung, an.

Sowohl bei den Silberkontakt-Verfahren wie auch bei den Metalloxid-Kontakt-Verfahren werden die heißen Reaktionsgase, die im Wesentlichen aus dem gebildeten Formaldehyd, nicht umgesetztem Methanol, Wasser, CO₂, CO, CH₄, Sauerstoff, Wasserstoff und Stickstoff bestehen, nach der Reaktion abgekühlt. Anschließend wird der entstandene Formaldehyd mit Wasser oder verdünnter Formaldehyd-Lösung ausgewaschen. Die Wäsche erfolgt üblicherweise mehrstufig in einer Absorptionskolonne im Gegenstrom. Die freiwerdende Absorptionswärme wird über einen Produktkreislauf in Wärmetauschern abgeführt und kann an anderer Stelle zur Beheizung eingesetzt werden.

Die Waschflüssigkeit wird im Allgemeinen auf dem Kopf der letzten Absorptionsstufe aufgegeben. Über die Menge an Waschflüssigkeit kann die Formaldehyd-Konzentration grob eingestellt werden.

Beim Verfahren mit vollständigem Methanol-Umsatz erhält man in der ersten Absorptionsstufe üblicherweise eine Formaldehyd-Lösung mit ca. 40 - 42 Gew.-%, bezogen auf das Gewicht der Lösung. In den weiteren Absorptionsstufen ist die Formaldehyd-Konzentration geringer.

Die wässrigen Formaldehyd-Lösungen erhalten noch Restmengen an Methanol (ca. 1 Gew.% beim Verfahren mit vollständigem Methanol-Umsatz). Beim Verfahren mit unvollständigem Methanol-Umsatz beträgt der Methanol-Gehalt der Absorptionslösung üblicherweise 5 - 15 Gew.%. Zur Abtrennung des Methanols wird die methanol-haltige Formaldehyd-Lösung abschließend destilliert. Man erhält dann üblicherweise Formaldehyd-Lösungen mit einer Konzentration von ca. 50 Gew.-% (45 - 55 Gew.-%) und 0,5 - 1 Gew.-% Methanol.

Beim Formox-Verfahren fallen in Abhängigkeit von der Menge an zugegebenem Waschwasser Formalin-Lösungen mit einer Konzentration von 37 - 55 Gew.-% Formaldehyd und 0,5 - 1,5 Gew.-% Methanol an. Dabei sind die Konzentrationsangaben in Gew.-% jeweils bezogen auf das Gewicht der wässrigen Formaldehyd-Lösung.

In Schritt b) erfolgt die online-analytische Ermittlung des Formaldehyd-Gehalts der wässrigen Formaldehyd-Lösung. Ausgangspunkt der vorliegenden Erfindung ist nämlich die Erkenntnis, dass sich beispielsweise die NIR-Absorptionsspektren von Formaldehyd / Wasser / Methanol-Gemischen überraschenderweise selbst bei kleineren Unterschieden in der Formaldehyd-Konzentration hinreichend voneinander unterscheiden, um die Formaldehyd-Konzentration in Formaldehyd / Wasser / Methanol-Gemischen aus dem Spektrum mit Hilfe eines chemometrischen Kalibrationsmodells mit der erforderlichen Genauigkeit, auch bei schwankenden Methanol-Gehalten, bestimmen zu können.

Die Bestimmung des Formaldehyd-Gehaltes der wässrigen Formaldehyd-Lösung in Schritt b) erfolgt so, dass online (d. h. wenigstens 1mal pro Minute) ein Spektrum der wässrigen Formaldehyd-Lösung mit einem optischen Sensor mittels Nah-Infrarot-(NIR)-Spektroskopie, Mittel-Infrarot-Spektroskopie oder Raman-Spektroskopie aufgenommen wird. Das gemessene Spektrum wird dann in ein chemometrisches Kalibrationsmodell eingegeben, das zuvor für Formaldehyd / Wasser / Methanol-Mischungen mit unterschiedlichen Konzentrationen und Verhältnissen der Einzelkomponenten erstellt wurde. Durch die Auswertung über das chemometrische Kalibrationsmodell erhält man die Konzentrationen an Formaldehyd, an Wasser und an Methanol in dem Formaldehyd / Wasser / Methanol-Gemisch (wässrige Formaldehyd-Lösung). Das chemometrische Kalibrationsmodell kann bevorzugt ein multivariantes Modell sein, z. B. basierend auf einen Partial Least Square-Algorithmus.

Eine geeignete Analysenmethode ist insbesondere die NIR-Spektroskopie. Ein besonderer Vorteil der NIR-Spektroskopie ist, dass die Aufnahme der Spektren online im Prozessstrom mittels Lichtleiter erfolgen kann. Die NIR-Strahlung wird hierbei mit Hilfe eines Lichtleiters über eine Sonde in den Prozessstrom eingestrahlt und dann nach Absorption über Lichtleiter zum Detektor des NIR-Spektrometers zurückgeführt. Die Detektion erfolgt im Nahen Infrarot-Bereich. Die hier auftretenden Oberton - und Kombinationsschwingungen werden mit Hilfe von statistischen Verfahren ausgewertet. Bevorzugte Bereiche für die Auswertung des Spektrums sind die Bereiche von 9000 cm⁻¹ bis 8000 cm⁻¹ und 6500 cm⁻¹ bis 5400 cm⁻¹, bevorzugt der Bereich von 6200 cm⁻¹ bis 5500 cm⁻¹. Die Messung kann hierbei direkt im Prozessstrom oder auch in einem Teilstrom erfolgen, der vom Prozessstrom abgezweigt wird.

Online im Sinne des erfindungsgemäßen Verfahrens bedeutet, dass wenigstens 1mal pro Minute, bevorzugt wenigstens 1mal pro 10 Sekunden, besonders bevorzugt wenigstens 1 mal pro Sekunde eine Messung erfolgt.

Der Fachmann ist mit der NIR-Spektroskopie und auch mit geeigneten Auswertemethoden wie chemometrischen Auswertemethoden beispielsweise aus den oben genannten Literaturstellen vertraut.

Bevorzugt wird in dem erfindungsgemäßen Verfahren in Schritt b) neben dem Formaldehyd-Gehalt auch noch simultan der Methanol-Gehalt der wässrigen Formaldehyd-Lösung bestimmt. Die oben genannten Analysen-Verfahren sind dazu in der Lage.

Die Messung des Formaldehyd-Gehalts und ggf. des Methanol-Gehalts in der wässrigen Formaldehyd-Lösung in Schritt b) kann an verschiedenen Stellen erfolgen. So kann die Messung beispielsweise im Ablauf der Absorptionskolonne, oder in der Leitung zu dem Behälter, in dem die wässrige Formaldehyd-Lösung aufgefangen oder gelagert wird, oder in einem Bypass eines solchen Behälters erfolgen.

In Schritt c) wird der ermittelte Formaldehyd-Gehalt mit einem vorgegebenen Sollwert verglichen. Bevorzugt wird der in der Konzentration einzustellende Formalin Mengenstrom gemessen. Zu diesem Formalin Mengenstrom wird dann falls erforderlich im für die Zielkonzentration notwendigen Verhältnis Wasser dazu gegeben. Dies erfolgt in Schritt d), der durchgeführt wird wenn der ermittelte Formaldehyd-Gehalt größer ist als der vorgegebene Sollwert für den Formaldehyd-Gehalt. Das einzustellende Verhältnis Formalin zu Wasser wird vorteilhaft durch Vergleich der ermittelten Ist-Formaldehyd-Konzentration (dem tatsächlichen gemessenen Formaldehyd-Gehalt) mit der spezifizierten Soll-Formaldehyd-Konzentration (dem gewünschten oder vorgegebenen Formaldehyd-Gehalt) berechnet.

Bevorzugt stellt man in Schritt a) einen solchen Formaldehyd-Gehalt der wässrigen Formaldehyd-Lösung ein, dass in Schritt d) in jedem Fall die Zugabe von Wasser erforderlich ist. Es ist jedoch auch der seltenere Fall denkbar, dass keine Korrektur mehr notwendig ist.

Es ist besonders vorteilhaft, wenn in Schritt b) neben dem Formaldehyd-Gehalt auch noch simultan der Methanol-Gehalt der wässrigen Formaldehyd-Lösung bestimmt wird. Denn wenn erhöhte Methanol-Gehalte in der wässrigen Formaldehyd-Lösung gemessen werden, besteht die Möglichkeit, in den Herstellprozess in Schritt a) einzugreifen und Produktions- oder Anlagenparameter für den Herstellprozess in Schritt a) anzupassen, so dass der Methanol-Gehalt in den bevorzugten Bereich kommt. Hierbei können auch Trends erkannt werden. So wird die Produktion von nichtspezifikationsgerechten Partien und von Partien mit eingeschränkter Produktqualität weitgehend vermieden.

Ein weiterer Vorteil ist, dass die manuelle Probenahme und die anschließende Labor-Analytik auch hinsichtlich der Methanol-Analytik entfallen.

Ein weiterer Vorteil ist die Möglichkeit der automatischen Prozessführung bei der Einstellung der Formaldehyd-Konzentration auf Grundlage der Konzentrationsinformationen für Formaldehyd und Methanol in der in Schritt a) erzeugten wässrigen Formaldehyd-Lösung. Dies ermöglicht auch die Einhaltung einer nahezu konstanten Produktqualität. Damit ist die Formalin-Herstellung, einschließlich der Einstellung des Formaldehyd-Gehalts der erzeugten wässrigen Formaldehyd-Lösungen in Schritt d), in einem vollständig kontinuierlichen Verfahren möglich.

Das erfindungsgemäße Verfahren kann auch vorteilhaft für die Herstellung von einzelnen Partien eingesetzt werden, wenn die in Schritt d) erhaltene wässrige Formaldehyd-Lösung anschließend in Behältern aufgefangen wird.

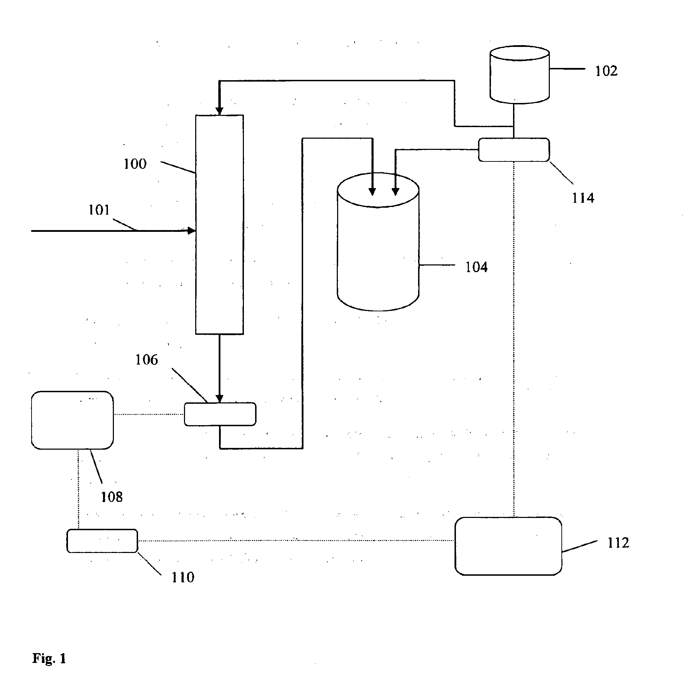

Die Erfindung wird im Folgenden anhand der Figur näher erläutert. Figur 1 zeigt das Blockdiagramm einer Ausführungsform des erfindungsgemäßen Verfahrens zur Herstellung einer wässrigen Formaldehyd-Lösung mit kontinuierlicher Wasser-Zugabe zur Einstellung der gewünschten Formaldehyd-Konzentration.

Das durch partielle Oxidation oder Dehydrierung von Methanol erzeugte Gemisch 101 enthaltend Formaldehyd sowie Wasser und Methanol, wird in einer Absorptionsvorrichtung 100 mit Wasser aus Tank 102 ausgewaschen. In der Leitung zwischen Absorptionsvorrichtung 100 und Vorratstank 104 ist eine optische Messzelle 106 eingebaut, die einen NIR (Nah Infrarot oder Near Infrared) -Sensor beinhaltet. Die Messzelle 106 ist vorzugsweise über einen Lichtwellenleiter mit dem Spektrometer 108 verbunden. Das Spektrometer 108 liefert ein Spektrum, das in ein chemometrisches Kalibrationsmodell 110 (umfassend eine Einrichtung, in der das Kalibrationsmodell implementiert und genutzt wird) eingegeben wird. Das chemometrische Kalibrationsmodell kann durch eine separate Auswerteeinheit, beispielsweise einen handelsüblichen Computer, realisiert werden. Alternativ kann das Spektrometer 108 selbst eine solche Auswerteeinheit für das Spektrum beinhalten.

Als Ergebnis der Analyse des gemessenen Spektrums gibt das chemometrische Kalibrationsmodell 110 den Ist-Gehalt an Formaldehyd und Methanol in der wässrigen Formaldehyd-Lösung an. Der Ist-Gehalt an Formaldehyd wird in einen Regler 112 eingegeben, in dem der gewünschte Soll-Formaldehyd-Gehalt hinterlegt ist. Aus einer Abweichung zwischen dem Ist - und dem Soll-Gehalt an Formaldehyd ermittelt der Regler 112 eine Stellgröße für die Pumpensteuerung der Pumpe 114 zur Zugabe des Wasserstroms aus dem Tank 102 in den Vorratstank 104. Die Messzelle kann beispielsweise auch in den Vorratstank 104 eingebaut werden, beispielsweise in eine Umpumpleitung.

Der Regler kann durch ein Prozessleitsystem der Formaldehyd-Herstellanlage realisiert werden. Wenn der Formaldehyd-Gehalt oder der Methanol-Gehalt außerhalb des für den Herstellprozess festgelegten Bereiches liegt, erfolgt bevorzugt eine Meldung.

Die durchgezogenen Linien in Figur 1 symbolisieren dabei den Stoffstrom der wässrigen Formaldehyd-Lösung bzw. des Wassers. Die gestrichelten Linien symbolisieren dagegen den Daten- und Informationsfluss, beispielsweise zwischen Messzelle und Spektrometer oder zwischen Kalibrationsmodell und Regler.

## Patentansprüche

1. Verfahren zur kontinuierlichen Herstellung einer wässrigen Formaldehyd-Lösung mit einem Formaldehyd-Gehalt von 25 - 56 Gew.-%, bezogen auf das Gewicht der wässrigen Formalde-hyd-Lösung, bei dem
a) Formaldehyd durch Oxidation von Methanol hergestellt wird und als wässrige Formaldehyd-Lösung erhalten wird, und
b) der Formaldehyd-Gehalt der wässrigen Formaldehyd-Lösung durch Messung eines Nah-Infrarot-, Mittel-Infrarot- oder Raman-Spektrums wenigstens 1mal pro Minute ermittelt wird, wobei das Spektrum in ein chemometrisches Kalibrationsmodell eingegeben wird, und
c) der ermittelte Formaldehyd-Gehalt mit einem vorgegebenen Sollwert verglichen wird, und, wenn der ermittelte Formaldehyd-Gehalt größer ist als der vorgegebene Sollwert für den Formaldehyd-Gehalt,
d) der gewünschte Formaldehyd-Gehalt der wässrigen Formaldehyd-Lösung durch Zugabe von Wasser eingestellt wird.

2. Verfahren nach Anspruch 1. bei dem der Formaldehyd-Gehalt der wässrigen Formaldehyd-Lösung durch Messung eines Nah-Infrarot-Spektrums ermittelt wird, das im Bereich von 6200 cm⁻¹ bis 5500 cm⁻¹ ausgewertet wird.

3. Verfahren nach einem der Ansprüche 1 bis 2, bei dem in Schritt b) zusätzlich der Methanol-Gehalt der wässrigen Formaldehyd-Lösung ermittelt wird.

4. Verfahren nach einem der Ansprüche 1 bis 3, bei dem in Schritt b) zusätzlich der Methanol-Gehalt der wässrigen Formaldehyd-Lösung durch Messung wenigstens 1mal pro Minute ermittelt wird.

5. Verfahren nach einem der Ansprüche 1 bis 4, bei dem der in Schritt b) ermittelte Gehalt an Formaldehyd und ggf. zusätzlich der in Schritt b) ermittelte Gehalt an Methanol für die Regelung des Herstellprozesses in Schritt a) verwendet wird.

## Claims

1. A continuous process for producing an aqueous formaldehyde solution having a formaldehyde content of from 25 to 56 wt.%, based on weight of aqueous formaldehyde solution, comprising:
a) oxidizing methanol to produce an aqueous formaldehyde solution, and
b) determining the formaldehyde content of the aqueous formaldehyde solution by measuring a near infrared spectrum, middle infrared spectrum or Raman spectrum at least once per minute, the spectrum being entered into a chemometric calibration model, and
c) comparing the formaldehyde content determined with a predetermined desired value, and, when the formaldehyde content determined is greater than the predetermined desired value for the formaldehyde content,
d) adding water to obtain the desired formaldehyde content in the aqueous formaldehyde solution.

2. The process of Claim 1 in which the formaldehyde content of the aqueous formaldehyde solution is determined by measuring a near infrared spectrum which is evaluated in the range from 6200 cm⁻¹ to 5500 cm⁻¹.

3. The process of either of Claims 1 and 2 in which the methanol content of the aqueous formaldehyde solution is also determined in step b).

4. The process of any of Claims 1 to 3 in which the methanol content of the aqueous formaldehyde solution is also determined in step b) by measuring at a rate of least once per minute.

5. The process of any of Claims 1 to 4 in which the formaldehyde content determined in step b) and optionally in addition the methanol content determined in step b) is used to control the production process in step a).

## Revendications

1. Procédé pour la préparation continue d'une solution aqueuse de formaldéhyde présentant une teneur en formaldéhyde de 25-56% en poids, par rapport au poids de la solution aqueuse de formaldéhyde, dans lequel
a) du formaldéhyde est préparé par oxydation de méthanol et est obtenu sous forme de solution aqueuse de formaldéhyde, et
b) la teneur en formaldéhyde de la solution aqueuse de formaldéhyde est déterminée par mesure d'un spectre infrarouge proche, infrarouge moyen ou Raman au moins une fois par minute, le spectre étant introduit dans un modèle de calibrage chimiométrique, et
c) la teneur en formaldéhyde déterminée est comparée à une valeur de consigne prédéfinie et, lorsque la teneur en formaldéhyde déterminée est supérieure à la valeur de consigne prédéfinie pour la teneur en formaldéhyde,
d) la teneur en formaldéhyde souhaitée de la solution aqueuse de formaldéhyde est réglée par addition d'eau.

2. Procédé selon la revendication 1, dans lequel la teneur en formaldéhyde de la solution aqueuse de formaldéhyde est déterminée par mesure d'un spectre infrarouge proche, qui est évalué dans la plage de 6200 cm⁻¹ à 5500 cm⁻¹.

3. Procédé selon l'une quelconque des revendications 1 à 2, dans lequel, dans l'étape b), la teneur en méthanol de la solution aqueuse de formaldéhyde est en outre déterminée.

4. Procédé selon l'une quelconque des revendications 1 à 3, dans lequel, dans l'étape b), la teneur en méthanol de la solution aqueuse de formaldéhyde est en outre déterminée par une mesure au moins une fois par minute.

5. Procédé selon l'une quelconque des revendications 1 à 4, dans lequel la teneur en formaldéhyde déterminée dans l'étape b) et le cas échéant en outre la teneur en méthanol déterminée dans l'étape b) est/sont utilisée(s) pour la régulation du procédé de préparation dans l'étape a).
